# EUROPEAN PATENT APPLICATION

(11) **EP 2 933 242 A1**
(43) Date of publication of application: **21.10.2015**
(21) Application number: 14164937.6
(22) Date of filing: 16.04.2014
(51) Int. Cl.: C07B 43/04, C07C 231/12

(54) **Process for manufacturing amino compounds**

(71) Applicant: BASF SE, 67056 Ludwigshafen (DE)
(72) Inventor: Wolf, Bernd, Dr., 67150 Niederkirchen (DE); Rack, Michael, Dr., 69214 Eppelheim (DE); Maywald, Volker, Dr., 67071 Ludwigshafen (DE); Goetz, Roland, Dr., 68809 Neulußheim (DE); Frassetto, Timo, Dr., 68199 Mannheim (DE)

(57) **Abstract**

The present invention relates to for manufacturing amino compounds of formula (I) 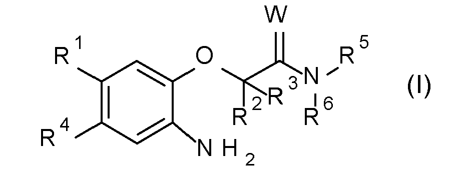 characterized in that nitro compounds of formula (II) are added to a reducing agent; wherein the substituents are defined according to the specification.

## Description

The invention relates to a process for manufacturing amino compounds and their use in a process for manufacturing benzoxazinones as well as in a process for manufacturing triazinonbenzoxazinones.

WO 13/092856 discloses a process for the preparation of amino compounds by reduction of a respective nitro compound, wherein the nitro compound is pre-charged and then the reduction agent is added. However said process has several disadvantages, as purity and yield of the amino compound are unsatisfying.

Hence, there is still room for improvement, specifically in view of economic aspects.

One task of the invention is to provide an efficient process for manufacturing amino compounds of formula (I).

Surprisingly it has been found that amino compounds of formula (I) can be obtained by reduction of nitro compounds of formula (II) wherein the nitro compound of formula (II) is added to a reducing agent.

It is therefore one object of the present invention to provide a process for manufacturing amino compounds of formula (I) 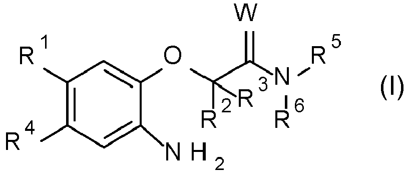 wherein
- R¹: is H or halogen;
- R²: is halogen;
- R³: is H or halogen;
- R⁴: is H, Hal, NH₂;
- R⁵, R⁶: are independently of each other C₁-C₆-alkyl, C₂-C₆-alkenyl, C₂-C₆-alkynyl, C₁-C₆-haloalkyl, C₁-C₆-cyanoalkyl, C₁-C₆-nitroalkyl, C₁-C₆-hydroxyalkyl, C₁-C₆-alkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₃-C₆-cycloalkyl, phenyl or benzyl, wherein the phenyl and the benzyl ring are independently of one another unsubstituted or substituted with1 to 5 substituents selected from the group consisting of halogen, C₁-C₆-alkyl or C₁-C₆-alkoxy, or R⁵ and R⁶ together with the N atom which they are attached to, represent a saturated or aromatic 3- to 6-membered ring, optionally containing 1 to 3 additional heteroatoms from the group O, S and N, with the ring optionally being substituted with 1 to 3 C₁-C₆-alkyl substituents; and
- W: is O or S;
characterized in that nitro compounds of formula (II) 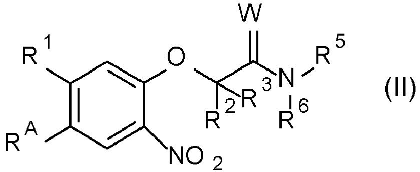
wherein R¹, R², R³, R⁵, R⁶ and W are defined as in formula (I); and
R^{A} is H, Hal, NH₂ or NO₂;
are added to a reducing agent.

The organic moieties mentioned in the definition of the variables according to the present invention, e.g. R¹ to R⁶, R^{A} are - like the term halogen - collective terms for individual enumerations of the individual group members.
The term halogen denotes in each case fluorine, chlorine, bromine or iodine. All hydrocarbon chains, i.e. all alkyl, can be straight-chain or branched, the prefix Cₙ-Cₘ denoting in each case the possible number of carbon atoms in the group.

Examples of such meanings are:
- C₁-C₄-alkyl: for example CH₃, C₂H₅, n-propyl, CH(CH₃)₂ n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-cyanoalkyl, C₁-C₆-nitroalkyl, C₁-C₆-hydroxyalkyl, C₁-C₆-alkyoxy-C₁-C₆-alkyl, amino-C₁-C₆-alkyl, (C₁-C₆-alkyl)amino-Ci-C₆-alkyl and di(C₁-C₆-alkyl)amino-C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₁-C₄-haloalkyl: a C₁-C₄-alkyl radical as mentioned above which is partially or fully substituted by fluorine, chlorine, bromine and/or iodine, for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, a C₁-C₃-haloalkyl radical as mentioned above, and also, for example, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;

- C₁-C₆-haloalkyl: C₁-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- C₃-C₆-cycloalkyl: monocyclic saturated hydrocarbons having 3 to 6 ring members, such as cyclopropyl, cyclobutyl, cyclopentyl and cyclohexyl;
- C₃-C₆-alkenyl: for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- C₂-C₆-alkenyl: C₃-C₆-alkenyl as mentioned above, and also ethenyl;
- C₃-C₆-alkynyl: for example 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
- C₂-C₆-alkynyl: C₃-C₆-alkynyl as mentioned above and also ethynyl;
- C₁-C₄-alkoxy: for example methoxy, ethoxy, propoxy, 1-methylethoxy butoxy, 1-methylpropoxy, 2-methylpropoxy and 1,1-dimethylethoxy;
- C₁-C₆-alkoxy: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxyand 1-ethyl-2-methylpropoxy.
- (C₁-C₄-alkyl)amino: for example methylamino, ethylamino, propylamino, 1-methylethyl-amino, butylamino, 1-methylpropylamino, 2-methylpropylamino or 1,1-dimethylethylamino;
- (C₁-C₆-alkyl)amino and also the (C₁-C₆-alkyl)amino moieties of (C₁-C₆-alkyl)amino-C₁-C₆-alkyl: (C₁-C₄-alkylamino) as mentioned above, and also, for example, pentylamino, 1-methylbutylamino, 2-methylbutylamino, 3-methylbutylamino, 2,2-dimethylpropylamino, 1-ethyl-propylamino, hexylamino, 1,1-dimethylpropylamino, 1,2-dimethylpropylamino, 1-methylpentylamino, 2-methylpentylamino, 3-methylpentylamino, 4-methylpentylamino, 1,1-dimethyl-butylamino, 1,2-dimethylbutylamino, 1,3-dimethylbutylamino, 2,2-dimethylbutylamino, 2,3-dimethylbutyl-amino 3,3-dimethylbutylamino, 1-ethylbutylamino, 2-ethylbutylamino, 1,1,2-trimethylpropylamino, 1,2,2-trimethyl-propylamino, 1-ethyl-1-methylpropylamino or 1-ethyl-2-methylpropylamino;
- di(C₁-C₄-alkyl)amino: for example N,N-dimethylamino, N,N-diethylamino, N,N-di(1-methylethyl)amino, N,N-dipropylamino, N,N-dibutylamino, N,N-di(1-methylpropyl)amino, N,N-di(2-methylpropyl)amino, N,N-di(1,1-dimethylethyl)amino, N-ethyl-N-methylamino, N-methyl-N-propylamino, N-methyl-N-(1-methylethyl)amino, N-butyl-N-methylamino, N-methyl-N-(1-methylpropyl)amino, N-methyl-N-(2-methylpropyl)amino, N-(1,1-dimethylethyl)-N-methylamino, N-ethyl-N-propylamino, N-ethyl-N-(1-methylethyl)amino, N-butyl-N-ethylamino, N-ethyl-N-(1-methylpropyl)amino, N-ethyl-N-(2-methylpropyl)amino, N-ethyl-N-(1,1-dimethylethyl)amino, N-(1-methylethyl)-N-propylamino, N-butyl-N-propylamino, N-(1-methylpropyl)-N-propylamino, N-(2-methylpropyl)-N-propylamino, N-(1,1-dimethylethyl)-N-propylamino, N-butyl-N-(1-methylethyl)amino, N-(1-methylethyl)-N-(1-methylpropyl)amino, N-(1-methylethyl)-N-(2-methylpropyl)amino, N-(1,1-dimethylethyl)-N-(1-methylethyl)amino, N-butyl-N-(1-methylpropyl)amino, N-butyl-N-(2-methylpropyl)amino, N-butyl-N-(1,1-dimethylethyl)amino, N-(1-methylpropyl)-N-(2-methylpropyl)amino, N-(1,1-dimethylethyl)-N-(1-methylpropyl)amino or N-(1,1-dimethylethyl)-N-(2-methylpropyl)amino;
- di(C₁-C₆-alkyl)amino and also the di(C₁-C₆-alkyl)amino moieties of di(C₁-C₆-alkyl)amino-C₁-C₆-alkyl: di(C₁-C₄-alkyl)amino as mentioned above, and also, for example, N-methyl-N-pentylamino, N-methyl-N-(1-methylbutyl)amino, N-methyl-N-(2-methylbutyl)amino, N-methyl-N-(3-methylbutyl)amino, N-methyl-N-(2,2-dimethylpropyl)amino, N-methyl-N-(1-ethylpropyl)amino, N-methyl-N-hexylamino, N-methyl-N-(1,1-dimethylpropyl)amino, N-methyl-N-(1,2-dimethylpropyl)amino, N-methyl-N-(1-methylpentyl)amino, N-methyl-N-(2-methylpentyl)amino, N-methyl-N-(3-methylpentyl)amino, N-methyl-N-(4-methylpentyl)amino, N-methyl-N-(1,1-dimethylbutyl)amino, N-methyl-N-(1,2-dimethylbutyl)amino, N-methyl-N-(1,3-dimethylbutyl)amino, N-methyl-N-(2,2-dimethylbutyl)amino, N-methyl-N-(2,3-dimethylbutyl)amino, N-methyl-N-(3,3-dimethylbutyl)amino, N-methyl-N- (1-ethylbutyl)amino, N-methyl-N-(2-ethylbutyl)amino, N-methyl-N-(1,1,2-trimethylpropyl)amino, N-methyl-N- (1,2,2-trimethylpropyl)amino, N-methyl-N-(1-ethyl-1-methylpropyl)amino, N-methyl-N- (1-ethyl-2-methylpropyl)amino, N-ethyl-N-pentylamino, N-ethyl-N-(1-methylbutyl)amino, N-ethyl-N-(2-methylbutyl)amino, N-ethyl-N-(3-methylbutyl)amino, N-ethyl-N-(2,2-dimethylpropyl)amino, N-ethyl-N-(1-ethylpropyl)amino, N-ethyl-N-hexylamino, N-ethyl-N-(1,1-dimethylpropyl)amino, N-ethyl-N-(1,2-dimethylpropyl)amino, N-ethyl-N-(1-methylpentyl)amino, N-ethyl-N-(2-methyl-pentyl)amino, N-ethyl-N-(3-methylpentyl)amino, N-ethyl-N-(4-methylpentyl)amino, N-ethyl-N-(1,1-dimethylbutyl)amino, N-ethyl-N-(1,2-dimethylbutyl)amino, N-ethyl-N-(1,3-dimethylbutyl)amino, N-ethyl-N-(2,2-dimethylbutyl)amino, N-ethyl-N-(2,3-dimethylbutyl)amino, N-ethyl-N-(3,3-dimethylbutyl)amino, N-ethyl-N-(1-ethylbutyl)amino, N-ethyl-N-(2-ethylbutyl)amino, N-ethyl-N-(1,1,2-trimethylpropyl)amino, N-ethyl-N-(1,2,2-trimethylpropyl)amino, N-ethyl-N-(1-ethyl-1-methylpropyl)amino, N-ethyl-N-(1-ethyl-2-methylpropyl)amino, N-propyl-N-pentylamino, N-butyl-N-pentylamino, N,N-dipentylamino, N-propyl-N-hexylamino, N-butyl-N-hexylamino, N-pentyl-N-hexylamino or N,N-dihexylamino;
- saturated or aromatic 3- to 6-membered ring optionally containing 1 to 3 additional heteroatoms selected from the group O, S and N:
   a monocyclic, saturated or aromatic cycle having three to six ring members which comprises apart from one nitrogen atom and carbon atoms optionally additionally one to three heteroatoms selected from the group O, S and N, for example:
      1-aziridinyl, 1-azetidinyl; 1-pyrrolidinyl, 2-isothiazolidinyl, 2-isothiazolidinyl, 1-pyrazolidinyl, 3-oxazolidinyl, 3-thiazolidinyl, 1-imidazolidinyl, 1,2,4-triazolidin-1-yl, 1,2,4-oxadiazolidin-2-yl, 1,2,4-oxadiazolidin-4-yl, 1,2,4-thiadiazolidin-2-yl, 1,2,4-thiadiazolidin-4-yl; 1-pyrrolyl, 1-pyrazolyl, 1-imidazolyl, 1,2,3-triazol-1-yl, 1,2,4-triazol-1-yl, 1-tetrazolyl; 1-piperidinyl, 1-hexahydropyridazinyl, 1-hexahydropyrimidinyl, 1-piperazinyl, 1,3,5-hexahydrotriazin-1-yl, 1,2,4-hexahydrotriazin-1-yl, tetrahydro-1,3-oxazin-1-yl, 1-morpholinyl.

The preferred embodiments of the invention mentioned herein below have to be understood as being preferred either independently from each other or in combination with one another.

According to a preferred embodiment of the invention preference is also given to the preparation of those amino compounds of formula (I), wherein the variables, either independently of one another or in combination with one another, have the following meanings:
- R¹: is preferably H or F; particularly preferred H;
is also preferably halogen, particularly preferred F or Cl, especially preferred F;
- R²: is preferably Cl or F, particularly preferred F;
- R³: is preferably H, Cl or F, particularly preferred H or F, especially preferred H;
is also preferably halogen, particularly preferred F or Cl, especially preferred F;
- R⁴: is preferably H or halogen, particularly preferred H;
is also preferably halogen or NH₂, particularly preferred halogen;
is also preferred H or NH₂, particularly preferred NH₂;
- R⁵ and R⁶: preferably are independently of each other C₁-C₆-alkyl, C₁-C₆-cyanoalkyl, C₁-C₆-hydroxyalkyl, C₁-C₆-alkoxy-C₁-C₆-alkyl, C₃-C₆-cycloalkyl, phenyl or benzyl,
wherein the phenyl and the benzyl ring are independently of one another unsubstituted or substituted with 1 to 3 substituents selected from the group consisting of halogen, C₁-C₆-alkyl or C₁-C₆-alkoxy,
or R⁵ and R⁶ together with the N atom which they are attached to, represent a saturated or aromatic 5- to 6-membered ring, optionally containing 1 additional heteroatom from the group O and N, with the ring optionally being substituted with 1 to 2 C₁-C₆-alkyl substituents;
particularly preferred are independently of each other C₁-C₄-alkyl, C₁-C₄-hydroxyalkyl, C₁-C₆-alkoxy-C₁-C₄-alkyl or benzyl,
wherein the benzyl ring is unsubstituted or substituted by 1 to 3 substituents selected from the group consisting of halogen, C₁-C₄-alkyl or C₁-C₄-alkoxy, especially preferred the benzyl ring is unsubstituted,
or R⁵ and R⁶ together with the N atom which they are attached to, represent a saturated 5-to 6-membered ring, optionally containing 1 additional oxygen atom, with the ring optionally being substituted with 1 to 2 C₁-C₆-alkyl substituents;
also particularly preferred are independently of each other C₁-C₆-alkyl;
- W: is preferably O,
is also preferably S.

Particular preference is given to the preparation of amino compounds of formula (Ia) corresponding to amino compounds of formula (I) wherein R¹, R² and R³ are F, R⁴ is NH₂, and W is O: 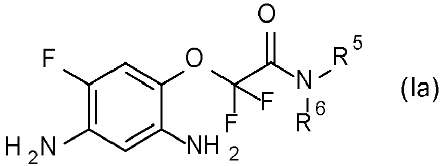 wherein the variables R⁵ and R⁶ have the meanings, in particular the preferred meanings, as defined above;
special preference to the the preparation of amino compounds of formulae (Ia.1) to (Ia.8) of Table A listed below, in which the variables R⁵ and R⁶ together have the meanings given in one row of Table A (compounds of formulae Ia-1 to Ia.8); and where the definitions of the variables R⁵ and R⁶ are of particular importance for the process and the compounds according to the invention not only in combination with one another but in each case also on their own:

**Table A**

| no. | R⁵ | R⁶ |
|---|---|---|
| Ia.1 | CH₃ | H |
| Ia.2 | CH₃ | CH₃ |
| Ia.3 | CH₃ | CH(CH₃)₂ |
| Ia.4 | C₂H₅ | C₂H₅ |
| Ia.5 | -CH₂-CH₂-CH₂-CH₂- | |
| Ia.6 | -CH₂-CH₂-CH₂-CH₂-CH₂- | |
| Ia.7 | -CH₂-CH₂-O-CH₂-CH₂- | |
| Ia.8 | -CH₂-CH₂-N(CH₃)-CH₂-CH₂- | |

With respect to the variables within the nitro compounds of formula (II), the particularly preferred embodiments correspond, either independently of one another or in combination with one another, to those of the variables of R¹, R², R³, R⁵, R⁶ and W of formula (I), or have, either independently of one another or in combination with one another, the following meanings:
- R^{A}: is preferably halogen, NH₂ or NO₂, particularly preferred NH₂ or NO₂, especially preferred NH₂;
is also preferably halogen or NH₂, particularly preferred halogen;
is also preferably halogen or NO₂, particularly preferred NO₂.

In the process according to the invention a nitro compound of formula (II) is added to a reducing agent to obtain the amino compounds of formula (I), 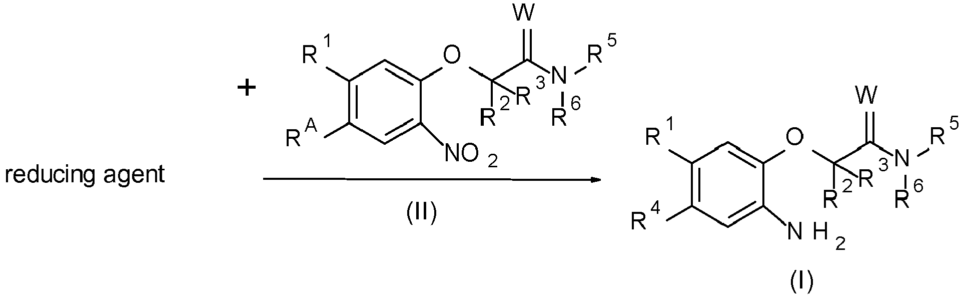 wherein the substituents are as herein defined.

The process according to the invention is generally carried out at a temperature in the range of from 0 °C to the boiling point of the solvents, preferably in the range from 20 to 80 °C, especially preferred in the range from 35 to 65 °C.

Examples of suitable reducing agents and conditions are known from the literature and can be found inter alia in Advanced Organic Chemistry (ed. J. March), 4th edition, Wiley-Interscience, NY 1992, p.1216 ff; or Organikum, 22nd edition, Wiley-VCH, Weinheim 2004, p. 626 ff.

Examples for reducing agents are molecular hydrogen, hydrazine, formic acid, ammonium formate, borane or borohydrides in combination with a homogeneous or heterogeneous catalyst, for example selected from metal salts of nickel, palladium, platinum, cobalt, rhodium, iridium, ruthenium or copper.
Specific examples for catalysts are palladium on charcoal, palladium on alumina, palladium on BaSO₄, platinum on charcoal, platinum on alumina, platinum(IV) oxide, Raney nickel, rhodium on alumina, ruthenium on alumina.

Further examples of suitable reducing agents are metals in their elemental form such as magnesium, iron, zinc, tin or metal salts such as tin(II) chloride in combination with an acid such as hydrochloric or acetic acid.

Further examples of suitable reducing agents are sulfur compounds like sodium hydrosulfite, rongalite, sodium sulfide, sodium hydrogen sulfide and ammonium polysulfide.

In a preferred embodiment of the process according to the invention the reducing agent is molecular hydrogen in combination with a homogeneous or heterogeneous hydrogenation catalyst.

Examples for hydrogenation catalysts are catalyst selected from the group consisting of metal salts of nickel, palladium, platinum, cobalt, rhodium, iridium, ruthenium or copper.

Specific examples for hydrogenation catalysts include palladium on charcoal, palladium on alumina, palladium on BaSO₄, platinum on charcoal, platinum on alumina, platinum(IV) oxide, Raney nickel, rhodium on alumina, ruthenium on alumina.

Preferred reducing agents are molecular hydrogen in combination with palladium on charcoal, palladium on alumina, palladium on BaSO₄, molecular hydrogen in combination with platinum on charcoal, platinum on alumina.

Especially preferred reducing agents are molecular hydrogen in combination with palladium on charcoal or platinum on charcoal.

A more preferred reducing agent is molecular hydrogen in combination with palladium on charcoal.
Also a more preferred reducing agent is molecular hydrogen in combination with platinum on charcoal.

The term reducing agent as used herein also includes mixtures of two or more, preferably two of the above compounds. Particular preference is given to the use of one reducing agent.

Accordingly, in a particularly preferred embodiment one reducing agent is employed in the process according to the invention.

The molar ratio of the nitro compound of formula (II) to the reducing agent is generally in the range of 1:2 to 1:15, preferably 1:2.5 to 1:10, more preferably 1:3 to 1:6.

Preferably the process according to the invention is carried out in a solvent.
Examples of suitable solvents are water, alcohols such as methanol, ethanol, 1-propanol, 2-propanol, 1-butanol, 2-butanol, iso-butanol, tert-butanol, 2-ethyl-hexanol, hexafluoroisopropanol; aromatic hydrocarbons such as benzene, toluene, ethylbenzene, cymene, xylenes, mesitylene, benzotrifluoride; esters such as methyl acetate, ethyl acetate, n-butyl acetate, isobutyl acetate; ethers such as di-n-butyl ether, tert-butyl methyl ether (TBME), tetrahydrofuran (THF), 1,4-dioxane, 1,2-dimethoxyethane; aliphatic hydrocarbons such as hexanes, cyclohexane; dipolar aprotic solvents such as N,N-dimethylformamide (DMF), N,N-dibutylformamide, N,N-dimethylacetamide (DMAC), 1-methyl-2-pyrrolidinone (NMP), 1,3-dimethyl-2-imidazolidinone (DMI), N,N'-dimethylpropylene urea (DMPU), dimethyl sulfoxide (DMSO), sulfolane.

Preferred solvents include methanol, toluene and xylenes.

More preferred solvents include methanol and toluene.

The term solvent as used herein also includes mixtures of two or more of the above solvents. Preference is given to the use of methanol/toluene mixtures or methanol/xylenes mixtures. Particular preference is given to the use of methanol/toluene mixtures.

In a preferred embodiment the reducing agent and the solvent are pre-charged in the reaction vessel and then the mixture is heated to a definite temperature, before the nitro compound of formula (II) is added.

In another preferred embodiment with the reducing agent being molecular hydrogen in combination with a catalyst, the catalyst and the solvent are pre-charged in the reaction vessel, the mixture is heated to a definite temperature, and then pressurized with hydrogen under stirring before the nitro compound of formula (II) is added.

In another preferred embodiment with the reducing agent being molecular hydrogen in combination with a catalyst, the catalyst and the solvent are pre-charged in the reaction vessel and then a solution of the nitro compound of formula (II) in a solvent is dosed to the pre-charged catalyst/solution mixture in that way, that the nitro compound of formula (II) is reduced immediately.

In another preferred embodiment with the reducing agent being molecular hydrogen in combination with a catalyst, the catalyst and the solvent are pre-charged in the reaction vessel and then a solution of the nitro compound of formula (II) in a solvent is dosed to the pre-charged catalyst/solvent mixture in that way, that the nitro compound of formula (II) is reduced immediately and the reaction temperature can be kept constant in a desired range.

In another preferred embodiment with the reducing agent being molecular hydrogen in combination with a catalyst, first the mixture of the catalyst and a solvent are stirred at a temperature in the range of from 20 to 80 °C, preferably 40 to 60 °C, in the presence of hydrogen at a pressure in the range of from 0 to 20 bar, preferably 0 to 10 bar; and then the nitro compound of formula (II) is added.

The process according to the invention is carried out under an elevated pressure of up to 20 bar, preferably up to 10 bar.

After completion or partial completion of the reaction, the respective mixture can be worked up by means of standard techniques. Examples thereof include extraction, filtration, aqueous work-up, distilling off solvents and/or other volatile compounds. These methods can also be combined with each other.

In a preferred embodiment the hydrogen source is removed and the catalyst is filtered off.

In another preferred embodiment the reaction mixture is brought to room temperature and the catalyst is filtered off. The solvent is partly or completely distilled off.

The product precipitates upon cooling, preferably to a temperature in the range of from 0 to 30 °C and/or adding water. The precipitated amino compound of formula (I) can be isolated by filtration.

The amino compounds of formula (I) are suitable intermediates for the synthesis of benzoxazinones e.g. as disclosed in WO 2013/092856.

Accordingly, in another preferred embodiment the catalyst is filtered off and the amino compound of formula (I) is not isolated, but the filtrate containing such amino compound of formula (I) is dosed immediately in another reactor to be used in one or more subsequent reaction step(s).

Preferably such filtration is conducted under inert conditions, especially under exclusion of oxygen.

According to another embodiment of the invention, the catalyst is filtered of and recycled.

The amino compounds of formula (I) are also suitable intermediates for the synthesis of triazinon-benzoxazinones, e.g. as disclosed in WO 2013/092856.

The nitro compounds of formula (II) necessary for the process according to the invention can be prepared by known methods, e.g. as described in WO 2013/092856.

The invention is illustrated by the following examples without being limited thereto or thereby.

### 1. Preparation of amino compounds of formula (I)

### Synthesis of 2,2-Difluoro-2-(2,4-diamino-5-fluoro-phenoxy)-N,N-dimethyl-acetamide

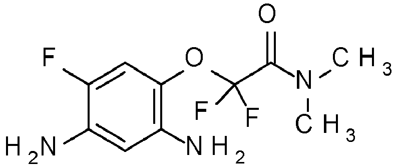

### Example 1.1:

29.8 g (0.007 mol) Pd/C (5% Pd in dry catalyst; 50% water content) and 579.4 g methanol were placed in the reaction vessel. The mixture was heated to 40°C and then pressurized with 2 bar H₂ under vigorous stirring.
A solution of 226.3 g (0.7 mol) 2,2-difluoro-2-(2,4-dinitro-5-fluoro-phenoxy)-N,N-dimethylacetamide in 895.7 g toluene was dosed within 3 hours. The transfer pipe was purged with 58 g toluene.
The temperature of the reaction mixture was held constantly at 39 - 41°C with cooling.
After completion of the reaction the pressure was released and the catalyst was filtered off through a pressure filter (1 bar nitrogen overpressure). The remaining catalyst was washed with 140 g methanol. The filtrate and wash methanol were combined and evaporated to dryness.

193.7 g of the desired product have been obtained having 90% purity determined by quantitative HPLC method as described below (t_{R} = 8.02 min; corresponding chemical yield 94% yield).

### Chromatographic conditions:

- Column:: Symmetry C18 5 µm 250 x 4.6 mm from Waters
- Wavelength:: 210 nm
- Eluent:: gradient of A (0.1 % by volume of H₃PO₄ in H₂O) and B (0.1 % by volume of H₃PO₄ in acetonitrile); starting with 10% B, then B rising from 10% to 50% within 10 min, then B rising from 50% to 90% within 20 min, then back to 10% B within 2 min, then 8 min 10% B
- Flow rate:: 1 ml/min
- Pressure:: approx. 160 bar
¹H NMR (DMSO-*d₆*, 400 MHz): δ (ppm) = 6.79 (d, 1 H); 6.16 (d, 1 H); 4.95 (bs, 2 H); 4.60 (bs, 2 H); 3.19 (s, 3 H); 2.95 (s, 3 H).

### Comparative example 1.2:

To a solution of 2,2-difluoro-2-(2,4-dinitro-5-fluoro-phenoxy)-*N*,*N* dimethyl-acetamide (22.0 g, 68.1 mmol) in toluene (200 g) Pd/C (10% Pd, dry catalyst, 0.7 g, 0.7 mmol) was added. Thereafter, methanol (80 g) was added and the mixture was stirred under an atmosphere of hydrogen (pressure of 0.1 bar) at 45 °C for 90 min. After completion of the reaction the pressure was released, the catalyst was filtered off and the filtrate was evaporated to dryness.
The product (17.3 g, 84% pure by NMR, 55.2 mmol, 81 % yield) was obtained as an off-white solid. If desired, the purity can be increased by chromatography (SiO₂, cyclohexane/EtOAc mixtures).
¹H NMR (DMSO-d₆, 500 MHz): δ (ppm) = 6.79 (d, J = 11.0 Hz, 1 H); 6.16 (d, J = 8.5 Hz, 1 H); 4.95 (bs, 2 H); 4.60 (bs, 2 H); 3.19 (s, 3 H); 2.96 (bs, 3 H).
¹³C NMR (DMSO-d₆, 125 MHz): δ (ppm) = 158.3 (t, J = 35 Hz); 141.7 (d, J = 278 Hz); 137.6; 134.9 (d, J = 14 Hz); 123.9 (d, J = 9 Hz); 115.8 (t, J = 272 Hz); 109.2 (d, J = 22 Hz); 102.0 (d, J = 4 Hz); 36.9; 36.2.

## Claims

1. A process for manufacturing amino compounds of formula (I), 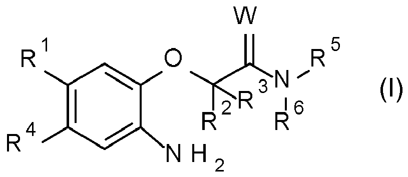 wherein
R¹ is H or halogen;
R² is halogen;
R³ is H or halogen;
R⁴ is H, Hal, NH₂;
R⁵, R⁶ are independently of each other C₁-C₆-alkyl, C₂-C₆-alkenyl, C₂-C₆-alkynyl, C₁-C₆-haloalkyl, C₁-C₆-cyanoalkyl, C₁-C₆-nitroalkyl, C₁-C₆-hydroxyalkyl, C₁-C₆-alkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₃-C₆-cycloalkyl, phenyl or benzyl, wherein the phenyl and the benzyl ring are independently of one another unsubstituted or substituted with1 to 5 substituents selected from the group consisting of halogen, C₁-C₆-alkyl or C₁-C₆-alkoxy,
or R⁵ and R⁶ together with the N atom which they are attached to, represent a saturated or aromatic 3- to 6-membered ring, optionally containing 1 to 3 additional heteroatoms from the group O, S and N, with the ring optionally being substituted with 1 to 3 C₁-C₆-alkyl substituents; and
W is O or S
**characterized in that** nitro compounds of formula (II) 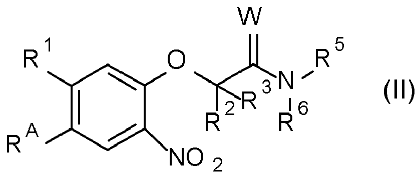
wherein R¹, R², R³, R⁵, R⁶ and W are defined as in formula (I);
R^{A} is H, Hal, NH₂ or NO₂;
are added to a reducing agent.

2. The process according to claim 1, wherein R¹ and R³ are halogen.

3. The process according to claims 1 or 2, wherein R⁴ is NH₂.

4. The process according to claims 1 to 3, wherein R^{A} is NO₂.

5. The process according to claims 1 to 4, wherein R⁵ and R⁶ are independently of one another C₁-C₆-alkyl.

6. The process according to claims 1 to 5 wherein the reducing agent is molecular hydrogen in combination with a homogeneous or heterogeneous hydrogenation catalyst.

7. The process according to claim 6, wherein the catalyst is palladium on charcoal or platinum on charcoal.

8. The process according to claim 6, wherein, before the nitro compounds of formula (II) are added, the catalyst and the solvent are pre-charged and the reaction vessel is pressurized with hydrogen.
